# EUROPÄISCHE PATENTANMELDUNG

(11) **EP 1 754 791 A1**
(43) Veröffentlichungstag der Anmeldung: **21.02.2007**
(21) Anmeldenummer: 06117961.0
(22) Anmeldetag: 27.07.2006
(51) Int. Cl.: C12P 7/62

(54) **Verfahren zur enzymatischen Herstellung von Chiralen, 1-acylierten 1,2 Diolen**

(30) Priorität: 16.08.2005 DE 102005038606
(71) Anmelder: Consortium für elektrochemische Industrie GmbH, 81379 München (DE)
(72) Erfinder: Pfaller, Rupert, Dr., 80639 München (DE); Reutter-Maier, Anneliese, 85614 Kirchseeon (DE); Schmid, Elida, 82515 Wolfratshausen (DE)
(74) Vertreter: Potten, Holger

(57) **Zusammenfassung**

Verfahren zur Herstellung einer chiralen Verbindung der Formel (I), 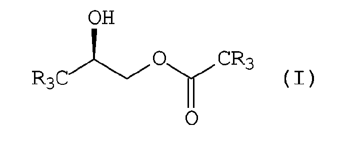 wobei R gleich oder verschieden ist und H, oder organischer Rest bedeutet, bei dem eine Biotransformationszusammensetzung, umfassend eine Verbindung der Formel (II), 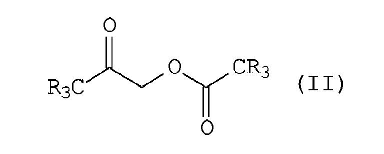 wobei R die genannte Bedeutung hat, eine Oxidoreduktase, einen Redox-Cofaktor und ein Cosubstrat, zur Reaktion gebracht wird, wobei die chirale Verbindung der Formel (I) entsteht und nachfolgend isoliert wird.

## Beschreibung

Die Erfindung betrifft ein Verfahren zur enzymatischen Herstellung eines chiralen, 1-acylierten Derivats von 1,2-Diolen der allgemeinen Formel (I), 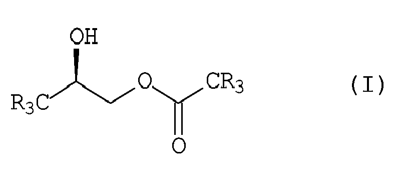 sowie von (R)-1-Acetoxy-2-Propanol (R-AcP) (R = H).

Optisch aktive Hydroxyverbindungen wie die chiralen Verbindungen der allgemeinen Formel (I) sind wertvolle Synthesebausteine, z. B. bei der Herstellung pharmazeutischer Wirkstoffe oder von Agrochemikalien. Diese Verbindungen sind durch klassische chemische Verfahren nur schwer herstellbar, denn die erforderlichen optischen Reinheiten für Anwendungen im pharmazeutischen oder agrochemischen Bereich sind auf diesem Wege nur schwer zu erreichen. Daher werden zur Herstellung chiraler Verbindungen im zunehmenden Maße biotechnologische Verfahren angewendet. Speziell Enzyme, die Carbonylverbindungen reduzieren können, finden wegen ihrer hohen Enantioselektivität vermehrt Verwendung.

Verbindungen der allgemeinen Formel (I) sind von synthetischem Interesse, da nur die OH-Gruppe in der 1-Position substituiert ist und die OH-Gruppe in der optisch aktiven 2-Position für die weitere Derivatisierung zur Verfügung steht. Alternative Methoden zur Herstellung dieser Verbindungen, z.B. die direkte Acylierung von 1,2-Diolen, führen vorzugsweise zur Substitution in 2-Position.

Enzyme aus der Klasse der Oxidoreduktasen, die zur Herstellung chiraler Verbindungen durch Reduktion prochiraler Carbonylverbindungen eingesetzt werden, werden mit dem Sammelbegriff Carbonylreduktase (in der Folge "CR") bezeichnet. In der Mehrzahl der Fälle ist das Produkt einer CR-Reaktion ein Alkohol. Es ist aber auch möglich, dass das Produkt einer CR-Reaktion ein Amin ist. Zu den Carbonylreduktasen zählen unter anderem Alkoholdehydrogenasen (in der Folge "ADH"), Aldo-Ketoreduktasen ("AKR"), Aldehydreduktasen, Glycerindehydrogenasen und die Fettsäuresynthase (bezeichnet als "FAS"). Aber auch Aminotransferasen oder Aminosäuredehydrogenasen (z. B. Threonindehydrogenase) sind zu den Carbonylreduktasen zu zählen. Diesem breiten Spektrum reduzierender Enzyme ist gemeinsam, dass sie die Elektronen für die Reduktion der Carbonylverbindung aus Redox-Cofaktoren in deren reduzierter Form, üblicherweise NADH oder NADPH, beziehen.

Die Redox-Cofaktoren NADH bzw. NADPH werden bei der enzymatischen Reduktion stöchiometrisch verbraucht, d.h. sie müssen entweder stöchiometrisch eingesetzt werden oder aber durch Oxidation eines Cosubstrats regeneriert werden (sog. Cofaktor-Regenerierung). Ein Cosubstrat ist dabei definiert als eine Verbindung, die als Reduktionsmittel enzymatisch oxidiert wird, wobei die dabei gewonnenen Elektronen auf NAD bzw. NADP übertragen werden und somit NADH bzw. NADPH regeneriert wird.

Für (S)-1-Acetoxy-2-Propanol (S-AcP) wurde eine Darstellung durch Biotransformation mit Zellen bzw. isolierten Enzymen aus der Bäckerhefe beschrieben (Ishihara et al. (1994), Bull. Chem. Soc. Jpn 67: 3314-3319, Ishihara et al. (1994), Tetrahedron Lett. 35, 4569-4570 und Ishihara et al. (1996), J. Ferment. Bioeng. 3: 266-268). Die geringen Raum-Zeitausbeuten, eine aufwändige Verfahrensführung durch Substratfütterung und eine hohe Einsatzmenge an Hefezellen führen dazu, dass die Verwendung von Hefezellen zur Darstellung von S-AcP im technischen Maßstab nicht praktikabel ist. Beispiel 7 beschreibt als Vergleichsbeispiel die alternative Herstellung von S-AcP mit dem Enzym T-ADH, einer S-selektiven ADH. Auch in diesem Fall sind die Raum-Zeitausbeuten vergleichsweise gering, so dass sich für den Fachmann aus den bekannten Verfahren zur Herstellung von S-AcP ein analoges Verfahren mit den für eine kostengünstige Herstellung von R-AcP erforderlichen hohen Raum-Zeitausbeuten mit einem R-selektiven Enzym nicht ableiten ließ. Dies gelang erst durch das in den Beispielen 1 bis 5 offenbarte Biotransformationsverfahren.

Aufgabe der Erfindung war es also, ein enzymatisches Verfahren zur Verfügung zu stellen, welches eine effiziente kostengünstige Herstellung von chiralen Verbindungen der Formel (I) ermöglicht.

Diese Aufgabe wird gelöst durch ein Verfahren, bei dem eine Biotransformationszusammensetzung, umfassend als Edukt eine Verbindung der Formel (II), 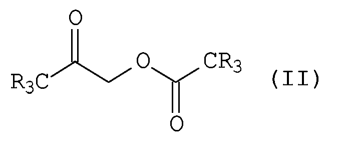 wobei R gleich oder verschieden ist und H, oder organischer Rest bedeutet, eine Oxidoreduktase, einen Redox-Cofaktor und ein Cosubstrat, zur Reaktion gebracht wird, wobei eine chirale Verbindung der Formel (I) 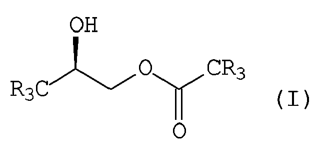 entsteht, wobei R die bereits genannte Bedeutung hat, und nachfolgend isoliert wird.

Vorzugsweise ist R gleich oder verschieden und bedeutet H oder C₁-C₂₀-Alkyl-, C₂-C₂₀-Alkenyl-, C₃-C₈-Cycloalkyl-, C₆-C₂₀-Aryl- oder C₅-C₂₀-Heteroarylrest, bei dem ein oder mehrere C-Atome durch Atome, ausgewählt aus der Gruppe B, N, O, Si, P und S, ersetzt sein können oder bei dem ein oder mehrere C-Atome substituiert sein können durch F, Cl, Br, J, C₃-C₈-Cycloalkyl, C₆₋C₂₀-Aryl, C₅-C₂₀-Heteroaryl, CN, NH₂, NO oder NO₂.

Besonders bevorzugt bedeutet R H oder C₁-C₂₀-Alkyl-, C₃-C₈₋Cycloalkyl-, C₆-C₂₀-Aryl- oder C₅-C₂₀-Heteroarylrest, bei dem ein oder mehrere C-Atome substituiert sein können durch F, Cl, C₃₋C₈-Cycloalkyl, C₆-C₂₀-Aryl oder C₅-C₂₀-Heteroaryl. Solche Verbindungen sind beispielsweise in Ishihara et al. (1994), Bull. Chem. Soc. Jpn 67: 3314-3319 offenbart.

Insbesondere bevorzugt hat R die Bedeutung von H.

Bei der Oxidoreduktase handelt es sich vorzugsweise um eine CR, die R-Spezifität besitzt.

Bei dem Redox-Cofaktor handelt es sich um eine Verbindung, die in ihrer reduzierten Form Elektronen zur Verfügung stellt, die in der enzymatischen Reaktion durch eine Oxidoreduktase auf das Edukt übertragen werden mit dem Resultat, dass ein erfindungsgemäßes Produkt entsteht. Der Redox-Cofaktor ist vorzugsweise ausgewählt aus Verbindungen der Gruppe NAD, NADP, (jeweils oxidierte Form des Cofaktors), NADH, NADPH (jeweils reduzierte Form des Cofaktors) und deren Salzen.

Die Redox-Cofaktoren in ihrer reduzierten Form, NADH bzw. NADPH, werden bei der enzymatischen Reduktion stöchiometrisch verbraucht, d.h. sie müssen entweder stöchiometrisch eingesetzt werden oder aber durch Oxidation eines Cosubstrats regeneriert werden (Cofaktor-Regenerierung). Der stöchiometrische Einsatz von NADH oder NADPH ist aufgrund des hohen Preises dieser Verbindungen unwirtschaftlich. Dieser Nachteil wird durch die Cofaktor-Regenerierung umgangen. Voraussetzung dafür sind ein billiges Cosubstrat (Reduktionsmittel) und ein Cofaktorreduzierendes Enzym. Erst die effiziente und billige Regenerierung des Redox-Cofaktors ermöglicht den technischen Einsatz biokatalytischer Reduktionsverfahren.

Ein Cosubstrat ist eine Verbindung, die als Reduktionsmittel enzymatisch oxidiert wird, wobei die dabei gewonnenen Elektronen auf NAD bzw. NADP übertragen werden und somit NADH bzw. NADPH regeneriert wird.

Wenn im erfindungsgemäßen Verfahren eine CR aus der Klasse der ADHs eingesetzt wird, so wird als Cosubstrat für die Cofaktor-Regenerierung ein Alkohol, vorzugsweise ein billiger Alkohol wie Isopropanol oder 2-Butanol eingesetzt. Es eignen sich aber auch alle anderen, vom 2-Butanol abgeleiteten höheren sekundären Alkohole. Damit wird in dieser Verfahrensvariante sowohl die stereoselektive Reduktion des Eduktes als auch die Cofaktor-Regenerierung vom gleichen Enzym, der ADH, besorgt.

Wenn im erfindungsgemäßen Verfahren eine CR, die nicht eine ADH ist, eingesetzt wird, so erfolgt die Cofaktor-Regenerierung mittels eines zweiten Enzyms. Dieses befindet sich ebenfalls im Reaktionsansatz. Die CR reduziert das Edukt stereoselektiv zum gewünschten Produkt, wobei der Cofaktor NADH bzw. NADPH verbraucht wird. Die Regenerierung des verbrauchten NADH bzw. NADPH wird durch ein zweites Enzym bewerkstelligt. Prinzipiell ist jedes Enzym zur Cofaktor-Regenerierung geeignet, das in einer enzymatischen Reaktion ein Substrat oxidiert und gleichzeitig NAD zu NADH bzw. NADP zu NADPH reduziert. Vorzugsweise wird ein Enzym verwendet, welches ein möglichst billiges Cosubstrat wie beispielsweise Glucose oder Ameisensäure bzw. dessen Salze oxidiert. Vorzugsweise wird als Enzym zur Cofaktor-Regenerierung ein Enzym aus der Gruppe Glucose-Dehydrogenase (GDH) und Formiat-Dehydrogenase (FDH) eingesetzt.

Bevorzugte Kombinationen von Enzym/Cosubstrat zur Cofaktorregenerierung sind die Kombination einer ADH mit einem Alkohol wie z.B. Isopropanol oder 2-Butanol oder die Kombination einer GDH mit Glucose.

Besonders bevorzugt ist die Kombination einer ADH mit einem Alkohol wie z.B. Isopropanol oder 2-Butanol.

Insbesondere bevorzugt ist die Kombination einer ADH mit Isopropanol.

Das erfindungsgemäße Verfahren ermöglicht es, durch enzymatische Reduktion eines Eduktes der Formel (II) bei hohen Raum-Zeitausbeuten und einem geringen Enzymeinsatz Verbindungen der Formel (I) mittels eines einfachen Batch-Verfahrens herzustellen.

Edukte der allgemeinen Formel (II) können nach dem Stand der Technik hergestellt werden, z. B durch Reaktion von 1-Cl-Ketonen, und hier besonders 1-Chloraceton, mit dem Salz einer Carbonsäure. So kann das besonders bevorzugte Edukt Acetoxyaceton auf diese Weise aus 1-Chloraceton und Kaliumacetat bzw. Natriumacetat hergestellt werden (Ishihara et al., (1994), Bull. Chem. Soc. Jpn 67: 3314-3319). Es eignet sich jedoch auch jedes andere Salz der Essigsäure zur Synthese von Acetoxyaceton.

Besonders bevorzugt ist die Herstellung von Acetoxyaceton aus 1-Chloraceton und Natriumacetat in einem kontinuierlichen Verfahren.

Als R-spezifische CRs werden vorzugsweise sekundäre ADHs, z. B. aus Stämmen der Gattung Lactobacillus wie die ADHs aus Lactobacillus brevis (LB-ADH), Lactobacillus kefir, Lactobacillus parabuchneri, Lactobacillus kandleri, Lactobacillus minor, oder es werden Fettsäuresynthetasen (FAS), besonders bevorzugt die FAS der Bäckerhefe oder aus Pichia pastoris, eingesetzt.

Bevorzugte R-selektive CRs sind ADHs der Gattung Lactobacillus.

Besonders bevorzugte R-selektive CR ist die LB-ADH.

Die zur enzymatischen Reduktion verwendeten CRs können hergestellt werden, indem man den Mikroorganismus kultiviert, aus dem die betreffende CR stammt. Dies geschieht jeweils in einer dem Fachmann bekannten Art und Weise. Das auf diese Weise hergestellte CR Enzym kann direkt in den Zellen des Produktionswirts verwendet werden, es kann aber auch nach Aufschluss der Zellen als Proteinextrakt bzw. nach entsprechender Aufarbeitung durch z. B. Säulenchromatographie als gereinigtes Protein eingesetzt werden.

Die Enzymproduktion der CRs kann mit einem sog. Expressionssystem auch in rekombinanter Form erfolgen. Dazu wird das für die betreffende CR kodierende Gen isoliert und, dem Stand der Technik entsprechend, in einen für die Proteinproduktion geeigneten Expressionsvektor kloniert. Nach Transformation des Expressionsvektors in einen geeigneten Wirtsorganismus wird ein Produktionsstamm isoliert. Mit diesem Produktionsstamm lässt sich die CR in an sich bekannter Weise, z. B. durch Fermentation, produzieren. Das auf diese Weise hergestellte CR Enzym kann dann direkt in den Zellen des Produktionswirts weiterverwendet werden oder aber nach Aufschluss der Zellen als Proteinextrakt bzw. nach entsprechender Aufarbeitung durch z. B. Säulenchromatographie als gereinigtes Protein.

Bevorzugt ist die Enzymproduktion der erfindungsgemässen CRs mit einem Expressionssystem in rekombinanter Form.

Für die Enzymproduktion sind bakterielle und eukaryontische Expressionssysteme geeignet. Wirtsorganismen für die Enzymproduktion sind vorzugsweise ausgewählt aus Escherichia coli, Stämmen der Gattung Bacillus, Hefen wie Pichia pastoris, Hansenula polymorpha oder Saccharomyces cerevisiae sowie Pilzen wie Aspergillus oder Neurospora, sie sind aber nicht auf die genannten Wirtsorganismen beschränkt.

Zu den bevorzugten Expressionssystemen zählen E. coli, Bacillus, Pichia pastoris, S. cerevisiae, Hansenula polymorpha oder Aspergillus.

Besonders bevorzugte Expressionssysteme für die Produktion des CR Enzyms sind E. coli, Pichia pastoris und S. cerevisiae.

Insbesondere bevorzugtes Expressionssystem ist E. coli.

Um einen möglichst kosteneffizienten Enzymeinsatz zu erreichen erfolgt die Enzymproduktion vorzugsweise durch Fermentation, besonders bevorzugt in einem Fed Batch Verfahren.

Vorzugsweise werden die Zellen aus der Fermentation dann direkt, im Fermentationsmedium suspendiert (Fermenterzellen) oder nach vorheriger Isolierung und anschließender Resuspension, in dem erfindungsgemäßen Verfahren weiterverwendet, so dass das erfindungsgemäße Verfahren als Ganzzellbiotransformation geführt wird. Es ist jedoch auch möglich, im erfindungsgemäßen Verfahren nach Aufschluss der Zellen den so erhaltenen Proteinextrakt oder nach entsprechender Aufarbeitung durch z. B. Säulenchromatographie das so erhaltene gereinigte Protein im erfindungsgemäßen Verfahren einzusetzen.

Besonders bevorzugt ist die Ganzzellbiotransformation, bei der zuerst die Enzymproduktion in einer rekombinanten Wirtszelle mittels Fermentation erfolgt und die Fermenterzellen anschließend direkt, im Fermentationsmedium suspendiert, in einer erfindungsgemäßen Biotransformation verwendet werden.

In der einfachsten Form umfasst eine erfindungsgemäße Biotransformationszusammensetzung (sog. Batchansatz) Fermenterzellen enthaltend ein CR-Enzym, als Edukt eine Verbindung der Formel (II), einen Redox-Cofaktor ausgewählt aus den Verbindungen NAD, NADH, NADP, NADPH, und deren Salzen, ein Cosubstrat ausgewählt aus der Gruppe Isopropanol, 2-Butanol und Glucose sowie bei Verwendung von Glucose als Cosubstrat eine GDH als Cofaktor-regenerierendes Enzym.

In einer abgewandelten Form des Verfahrens werden eine oder mehrere der genannten Komponenten der Biotransformationszusammensetzung kontinuierlich oder diskontinuierlich zudosiert (sog. Fed Batchansatz).

Bevorzugtes Verfahren ist der Batchansatz.

Vorzugsweise enthält eine erfindungsgemäße Biotransformationszusammensetzung zwischen 1 %(v/v) und 40 % (v/v) einer aus der Fermentation erhaltenen Suspension von Fermenterzellen, mit einem Biomasseanteil von 0,05 - 2 % (w/v), enthaltend ein CR-Enzym. Der Biomasseanteil ist dabei definiert als Trockenbiomasse, die man erhält, wenn die Fermenterzellen, z. B. in einem Trockenschrank bei 105°C, bis zur Gewichtskonstanz getrocknet werden.

Besonders bevorzugt enthält die Zusammensetzung zwischen 5 % (v/v) und 30 % (v/v) einer aus der Fermentation erhaltenen Suspension von Fermenterzellen, enthaltend ein CR-Enzym, mit einem Biomasseanteil von 0,25 - 1,5 % (w/v).

Insbesondere bevorzugt enthält die Zusammensetzung zwischen 10 % (v/v) und 25 % (v/v) einer aus der Fermentation erhaltenen Suspension von Fermenterzellen, enthaltend ein CR-Enzym, mit einem Biomasseanteil von 0,5 - 1,25 % (w/v).

Eine erfindungsgemäße Biotransformationszusammensetzung zeichnet sich ferner dadurch aus, dass der Anteil an Edukt der Formel (II) zwischen 10 %(w/v) und 60 % (w/v) des Gesamtansatzes beträgt.

Bevorzugt liegt der Anteil an erfindungsgemäßem Edukt der Formel (II) zwischen 20 %(w/v) und 50 % (w/v) des Gesamtansatzes.

Insbesondere bevorzugt ist eine Zusammensetzung, bei der der Anteil an Edukt der Formel (II) zwischen 30 %(w/v) und 45 % (w/v) des Gesamtansatzes beträgt.

Eine erfindungsgemäße Biotransformationszusammensetzung zeichnet sich auch dadurch aus, dass der Anteil an Cosubstrat im Falle von Isopropanol oder 2-Butanol vorzugsweise zwischen 10 % (w/v) und 50 % (w/v) des Gesamtansatzes beträgt.

Besonders bevorzugt beträgt der Anteil an Cosubstrat im Falle von Isopropanol oder 2-Butanol zwischen 20 % (w/v) und 45 % (w/v) des Gesamtansatzes.

Insbesondere bevorzugt beträgt der Anteil an Cosubstrat im Falle von Isopropanol oder 2-Butanol zwischen 30 %(w/v) und 40 % (w/v) des Gesamtansatzes.

Wird Glucose als Cosubstrat verwendet, enthält die Zusammensetzung Glucose vorzugsweise in einer Konzentration von 20 % (w/v) bis 65 % (w/v) bezogen auf den Gesamtansatz.

Eine erfindungsgemäße Biotransformationszusammensetzung umfasst vorzugsweise den Redox-Cofaktor in einer Konzentration zwischen 10 µM und 200 µM, besonders bevorzugt zwischen 20 µM und 150 µM, insbesondere bevorzugt zwischen 40 µM und 100 µM.

Das erfindungsgemäße Verfahren wird vorzugsweise bei einer Temperatur von 3°C bis 70°C, bevorzugt von 5°C bis 50°C, insbesondere bevorzugt von 15°C bis 40°C durchgeführt.

Vorzugsweise wird es in einem pH-Bereich von 5 bis 9, bevorzugt von 5,5 bis 8, insbesondere bevorzugt von 6 bis 7,5 durchgeführt. Vorzugsweise ist der Ansatz zur Konstanthaltung des pH Wertes gepuffert. Vorzugsweise erfolgt eine pH-Kontrolle über eine Titrationsvorrichtung, gekoppelt an ein pH-Messgerät (sog. pH-Stat Methode).

Die Reaktionsdauer des erfindungsgemäßen Verfahrens beträgt vorzugsweise 5 h bis 100 h, besonders bevorzugt 10 h bis 60 h, insbesondere bevorzugt 15 h bis 40 h.

Unter den genannten Bedingungen werden Edukte der allgemeinen Formel (II) zu > 80 %, bevorzugt > 90 %, insbesondere bevorzugt > 93 % zu einem Produkt der allgemeinen Formel (I) umgesetzt.

Das erfindungsgemäße Verfahren ermöglichte es erstmals, Verbindungen der allgemeinen Formel (I), insbesondere R-AcP, herzustellen.

Insbesondere wurde überraschend gefunden, dass Edukte der allgemeinen Formel (II) und darunter bevorzugt Acetoxyaceton in einer Dosierung von 20 - 43 % (w/v) in einer Reaktionszeit von 24 h mit einer Umsatzrate von mehr als 90 % in ein Produkt der allgemeinen Formel (I), darunter bevorzugt R-AcP, mit einer E-nantiomerenreinheit ee von 100% umgesetzt werden konnten, wobei gleichzeitig die Dosierung CR-haltiger Zellen, ausgedrückt in Trockenbiomasse Fermenterzellen, nicht mehr als 1 % (w/v) vom Ansatzvolumen betrug. Die beobachtete Effizienz und die für eine Biotransformation unerwartet hohen Raum-Zeitausbeuten des erfindungsgemässen Biotransformationsverfahrens waren nach dem Stand der Technik nicht zu erwarten.

Die Extraktion des Produktes erfolgt nach an sich bekannten Methoden, vorzugsweise mit einem mit Wasser nicht mischbaren, organischen Lösungsmittel. Die Extraktion kann dabei diskontinuierlich (batchweise) oder kontinuierlich erfolgen. Wie dem Fachmann bekannt, wird dabei eine Temperatur eingestellt, dass eine optimale Extraktion des Produktes aus der wässrigen Phase gewährleistet ist. Vorzugsweise erfolgt die Extraktion bei einer Temperatur von 10 bis 70°C.

Alternativ ist auch die direkte Produktgewinnung durch Destillation möglich.

Als organische Lösemittel sind alle mit Wasser nicht mischbaren Lösemittel geeignet, die eine Verbindung der Formel (I) aus einer wässrigen Phase extrahieren können.

Bevorzugt werden organische Lösemittel, ausgewählt aus der Gruppe der Ester, Ether, Alkane und Aromaten, verwendet.

Besonders bevorzugt werden Ethylacetat, Methylacetat, Propylacetat, Isopropylacetat, Butylacetat, Tertbutylacetat, Diethylether, Diisopropylether, Dibutylether und Methyl-Tertbutylether (MTBE), Pentan, Hexan, Heptan, Toluol oder deren Mischungen verwendet.

Insbesondere bevorzugte Lösungsmittel sind MTBE, Ethylacetat und Butylacetat.

Nach der Abtrennung der organischen Extraktionsphase wird diese vorzugsweise destillativ aufgearbeitet, wobei eine Anreicherung des Reaktionsproduktes erreicht und die teilweise bis vollständige Abtrennung von Nebenprodukten vom Extraktionslösemittel bewirkt wird und dieses erneut zur Extraktion eingesetzt werden kann.

Durch Aufreinigung der organischen Extraktionslösung enthaltend das Rohprodukt, beispielsweise mittels Feindestillation, erhält man das gewünschte Endprodukt. Das Endprodukt wird typischerweise in einer Ausbeute >70 %, bevorzugt >80 %, besonders bevorzugt >90 %, jeweils bezogen auf die eingesetzte Menge des Eduktes (II), erhalten. Es besitzt einen Enantiomerenüberschuss von bevorzugt ee >90 %, besonders bevorzugt ee >97 % insbesondere bevorzugt ee = 100 %.

Die folgenden Beispiele dienen zur Beschreibung der Erfindung:

### 1. Beispiel:

### Herstellung von LB-ADH durch Fermentation

Das Enzym LB-ADH, sein Gen und die rekombinante Produktion von LB-ADH in E. coli sind in EP796914 offenbart. Es wurde das in E. coli transformierte und in EP796914 offenbarte Plasmid pADH-1 verwendet. Alternativ kann das Enzym als aus rekombinanten E. coli hergestellter Rohextrakt kommerziell von der Fa. Jülich Fine Chemicals GmbH bezogen werden.

### Fermentation von LB-ADH produzierenden E. coli:

Herstellung eines Inokulums für die Fermentation:
1. Vorkultur von E. coli pADH-1 in LBamp Medium. Die Anzucht erfolgte für 7 bis 8 h auf einem Orbitalschüttler (Infors) bei 120 rpm und 30°C.
   LBamp Medium enthielt Pepton vegetable (Oxoid) 10 g/l; Hefeextrakt (Oxoid) 5 g/l; NaCl 5 g/l und Ampicillin 0,1 g/l.
2. Vorkultur: 100 ml SM3amp Medium wurden in einem 1 l Erlenmeyerkolben mit 1,3 ml Schüttelkultur beimpft. Die Anzucht erfolgte für 16 - 18 h bei 30°C und 120 rpm auf einem Orbitalschüttler bis zu einer Zelldichte OD_{600/}ml von 7 - 10. 100 ml der Vorkultur wurden zum Beimpfen von 1 l Fermentermedium verwendet.
   SM3amp Medium enthielt Pepton vegetable (Oxoid) 5 g/l; Hefeextrakt (Oxoid) 2,5 g/l; NaCl 0,1 g/l; Ammoniumsulfat 5 g/l; KH₂PO₄ 3 g/l; K₂HPO₄ 12 g/l; Glucose 5 g/l; MgSO₄ x 7 H₂O 0,3 g/l; CaCl₂ x 2 H₂O 14,7 mg/l; FeSO₄ x 7 H₂O 2 mg/l; Natriumcitrat x 2 H₂O 1 g/l; Vitamin B1 5 mg/l; Spurenelementemix 1 ml/l und Ampicillin 0,1 g/l.

Der Spurenelementemix hatte die Zusammensetzung H₃BO₃ 2,5 g/l; CoCl₂ x 6 H₂O 0,7 g/l; CuSO₄ x 5 H₂O 0,25 g/l; MnCl₂ x 4 H₂O 1,6 g/l; ZnSO₄ x 7 H₂O 0,3 g/l und Na₂MoO₄ x 2 H₂O 0,15 g/l.

Die Fermentationen wurden in Biostat CT Fermentern der Firma Sartorius BBI Systems GmbH durchgeführt. Fermentationsmedium war FM2amp. Die Fermentation erfolgte im sog. Fed-Batch Modus.

FM2amp Medium enthielt Glucose 20 g/l; Pepton vegetable (Oxoid) 5 g/l; Hefeextrakt (Oxoid) 2,5 g/l; Ammoniumsulfat 5 g/l; NaCl 0,5 g/l; FeSO₄ x 7 H₂O 75 mg/l; Na₃Citrat x 2 H₂O 1 g/l; CaCl₂ x 2 H₂O 14,7 mg/l; MgSO₄ x 7 H₂O 0,3 g/l; KH₂PO₄ 1,5 g/l; Spurenelementemix 10 ml/l; Vitamin B1 5 mg/l und Ampicillin 0,1 g/l. Der pH des FM2amp Mediums wurde vor Beginn der Fermentation auf 7,0 eingestellt.

1 l FM2amp wurde mit 100 ml Inokolum beimpft. Fermentationstemperatur war 30°C. pH der Fermentation war 7,0 und wurde mit den Korrekturmitteln 25 % NH₄OH bzw. 6 N H₃PO₄ konstant gehalten. Die Belüftung erfolgte mit Pressluft bei einem konstanten Durchfluss von 5 slpm (Standard Liter pro Minute). Der Sauerstoffpartialdruck pO₂ wurde auf 50% Sättigung eingestellt. Die Regulierung des Sauerstoffpartialdruckes erfolgte über die Rührgeschwindigkeit (Rührerdrehzahl 450 - 1.300 rpm). Zur Kontrolle der Schaumbildung wurde Struktol J673 (20-25 % v/v in Wasser) verwendet.

Im Verlauf der Fermentation wurde der Glucoseverbrauch durch off-line Glucose-Messung mit einem Glucose-Analysator der Fa. YSI bestimmt. Sobald die Glukosekonzentration der Fermentationsansätze ca. 5 g/l betrug (5 - 6 h nach Inokulation), wurde die Zudosierung einer 60% w/w Glucose Feedlösung gestartet. Die Flußrate des Feeds wurde so gewählt, dass während der Produktionsphase eine Glukosekonzentration von 1 - 5 g/l eingehalten werden konnte.

Induktion der LB-ADH Produktion erfolgte durch Zugabe von IPTG (Stammlösung 100 mM) in einer Konzentration von 0,4 - 0,8 mM, sobald das Zellwachstum im Fermenter eine OD₆₀₀/ml von 50 - 60 erreicht hatte. Die gesamte Fermentationsdauer betrug 32 h. Nach Beendigung der Fermentation wurde die Fermenterbrühe (Trockenbiomasse 50 g/l) in Aliquots zu je 100 ml eingefroren.

### 2.Beispiel:

### Herstellung eines LB-ADH Rohextrakts

2 l Zellsuspension aus der Fermentation von E. coli pADH-1 (siehe 1. Beispiel) wurden zentrifugiert (15 min 8.000 rpm bei 4°C, GS 3-Rotor, Sorvall Zentrifuge). Das Sediment wurde in 500 ml 50 mM Kaliumphosphat, pH 7,0, 1 mM MgCl₂ resuspendiert und durch drei Passagen durch einen Hochdruckhomogenisator (NS1001L Panda 2K der Fa. Niro Soavi) bei 800 bar Druck aufgeschlossen. Das Homogenat wurde zentrifugiert (30 min 8.000 rpm bei 4°C, GS 3-Rotor, Sorvall Zentrifuge). Der Überstand ergab einen LB-ADH Rohextrakt von 535 ml Volumen. Die Bestimmung der LB-ADH Aktivität ergab eine Volumenaktivität von 1.300 U/ml bzw. eine spezifische Aktivität von 108 U/mg Protein im Rohextrakt.

Spektralphotometrische Bestimmung der LB-ADH Aktivität:
Der Messansatz von 1 ml Volumen zur photometrischen Bestimmung von LB-ADH Aktivität war zusammengesetzt aus Messpuffer (0,1 M Kaliumphosphat, pH 7,0, 0,1 M NaCl, 1 mM MgCl₂), 3 µl Substrat 4-Cl-Acetessigsäureethylester, 0,2 mM NADPH und LB-ADH-haltigem Zellextrakt. Messtemperatur war 25°C. Die Reaktion wurde gestartet durch Zugabe des LB-ADH Zellextrakts und die Extinktionsabnahme infolge des Verbrauchs von NADPH bei einer Wellenlänge von 340 nm gemessen (Extinktionskoeffizient von NADPH: E = 0,63 x 10⁴ 1 x Mol⁻¹ x cm⁻¹). Ein Unit LB-ADH-Aktivität ist definiert als der Verbrauch von 1 pmol NADPH/min unter Testbedingungen.

Zur Bestimmung der spezifischen Aktivität wurde die Proteinkonzentration der Zellextrakte in an sich bekannter Weise mit dem sog. "BioRad Proteinassay" der Fa. BioRad bestimmt.

### 3. Beispiel:

### Gaschromatographische Analytik

### GC-MS Analyse:

Zur Herstellung einer racemischen Referenzsubstanz von 1-Acetoxy-2-Propanol wurde Acetoxyaceton (CAS-RN 529-20-1) in an sich bekannter Weise durch Behandlung mit NaBH₄ unselektiv reduziert. Das Produktgemisch, das neben dem Hauptprodukt 1-Acetoxy-2-Propanol in kleinen Mengen auch das Umlagerungsprodukt 2-Acetoxy-1-Propanol enthielt, wurde durch GC-MS, dem Stand der Technik entsprechend ohne chirale Trennung analysiert. Die bei folgenden Retentionszeiten bestimmten Molmassen waren:
Acetoxyaceton: 3,71 min, Molmasse 116.
1-Acetoxy-2-Propanol: 3,97 min, Molmasse 118.
2-Acetoxy-1-Propanol: 4,17 min, Molmasse 118.

### Chirale GC:

Verwendet wurde ein Gaschromatograph 6890N der Fa. Agilent incl. Flammenionisationsdetektor, der mit einer CP-Chirasil-Dex-CB Säule der Fa. Varian (25 m x 0,25 mm) zur chiralen Trennung ausgerüstet war.

Zur gaschromatochraphischen Trennung wurde ein Temperaturgradient von 100°C - 140°C mit einer Gradientensteilheit von 2°C/min, gefolgt von einem Temperaturgradienten von 140°C - 170°C mit einer Gradientensteilheit von 10°C/min eingestellt. Retentionszeiten unter diesen Bedingungen waren:
Acetoxyaceton: 4,4 min.
(R)-1-Acetoxy-2-Propanol: 6,2 min.
(S)-1-Acetoxy-2-Propanol: 6,4 min.
(R)-2-Acetoxy-1-Propanol: 7,8 min.
(S)-2-Acetoxy-1-Propanol: 8,7 min.

### 4. Beispiel:

### Produktnachweis durch Verseifung von (R)-1-Acetoxy-2-Propanol zum (R)-Propylenglykol und Analyse durch chirale GC

100 ml Reaktionsgemisch aus einem Ansatz, wie im 5. Beispiel beschrieben, wurde dreimal mit je 100 ml MTBE extrahiert und das Lösungsmittel der vereinigten Extraktionsphasen in einem Rotationsverdampfer abdestilliert.

Das dabei gewonnene Rohprodukt von (R)-1-Acetoxy-2-Propanol wurde durch Behandlung mit NaOH verseift. Der Ansatz zur Verseifung enthielt 1 ml Rohprodukt, 1,5 ml H₂O, 1 ml 10 M NaOH und 7,5 ml Methanol. Nach 2 h Inkubation bei 50°C wurde 1 ml des Ansatzes mit 1 ml MTBE extrahiert und entstandenes Propylenglykol durch chirale GC analysiert.

Verwendet wurde ein dem Stand der Technik entsprechender Gaschromatograph 6890N der Fa. Agilent incl. Flammenionisationsdetektor, der mit einer CP-Chirasil-Dex-CB Säule der Fa. Varian (25 m x 0,25 mm) zur chiralen Trennung ausgerüstet war.

Zur gaschromatographischen Trennung wurde ein Temperaturgradient von 65°C - 170°C mit einer Gradientensteilheit von 15°C/min eingestellt. Referenzsubstanzen für (R)- und (S)-Propylenglykol sind kommerziell erhältlich (Sigma Aldrich). Unter den Bedingungen der chiralen GC ergaben sich folgende Retentionszeiten:
(S)-Propylenglykol: 18,1 min.
(R)-Propylenglykol: 18,4 min.

Das nach Verseifung aus der Biotransformation gewonnene (R)-Propylenglykol erschien als einzelner Peak. Aufstockexperimente mit (R)- bzw. (S)-Propylenglykol Referenzsubstanzen bestätigten, dass die Biotransformation von Acetoxyaceton selektiv zu (R)-1-Acetoxy-2-Propanol führte. Nebenprodukt des (S)-Enantiomers konnte nicht nachgewiesen werden. Der Enantiomerenüberschuss ee betrug 100 %.

Bei einer analog durchgeführten Analyse, bei der Rohprodukt aus der Biotransformation mit T-ADH gewonnen worden war (siehe 7. Beispiel), wurde entsprechend (S)-Propylenglykol mit einem ee von 100 % nachgewiesen.

### 5. Beispiel:

### Biotransformationen mit LB-ADH Zellen

20 % Acetoxyaceton-Dosierung: Ein Reaktionsansatz war zusammengesetzt aus 20 ml (21,5 g) Acetoxyaceton, 40 ml (31,4 g) Isopropanol, 12 ml LB-ADH Zellen (Fermenterbrühe wie im 1. Beispiel beschrieben), 50 µM NADP und 28 ml KPi-Puffer. Die Zusammensetzung von KPi-Puffer war 0,1 M Kaliumphosphat, pH 7,0, 0,1 M NaCl, 1 mM MgCl₂. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 99 %. Der Enantiomerenüberschuss ee des Produktes (R)-1-Acetoxy-2-Propanol betrug 100 %.

30 % Acetoxyaceton-Dosierung: Ein Reaktionsansatz war zusammengesetzt aus 30 ml (32,3 g) Acetoxyaceton, 40 ml (31,4 g) Isopropanol, 12 ml LB-ADH Zellen, 50 µM NADP und 18 ml KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 95 %. Der Enantiomerenüberschuss ee des Produktes (R)-1-Acetoxy-2-Propanol betrug 100 %.

40 % Acetoxyaceton-Dosierung: Ein Reaktionsansatz war zusammengesetzt aus 40 ml (43 g) Acetoxyaceton, 40 ml (31,4 g) Isopropanol, 12 ml LB-ADH Zellen, 50 µM NADP und 8 ml KPi-Puffer. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 89 %. Der Enantiomerenüberschuss ee des Produktes (R)-1-Acetoxy-2-Propanol betrug 100 %.

40 % Acetoxyaceton-Dosierung: Ein Reaktionsansatz war zusammengesetzt aus 40 ml (43 g) Acetoxyaceton, 40 ml (31,4 g) Isopropanol, 20 ml LB-ADH Zellen und 50 µM NADP. pH der Reaktion war 7,0. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 95 %. Der Enantiomerenüberschuss ee des Produktes (R)-1-Acetoxy-2-Propanol betrug 100%.

### 6. Beispiel:

### Biotransformation mit LB-ADH Rohextrakt

30 % Acetoxyaceton-Dosierung: Ein Reaktionsansatz war zusammengesetzt aus 30 ml (32,3 g) Acetoxyaceton, 40 ml (31,4 g) Isopropanol, 30 ml Extrakt aus LB-ADH Zellen (39.000 U LB-ADH, siehe 2. Beispiel) und 50 µM NADP. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 90 %. Der Enantiomerenüberschuss ee des Produktes (R)-1-Acetoxy-2-Propanol betrug 100 %.

### 7. Beispiel (Vergleichsbeispiel):

### Biotransformation mit T-ADH Enzym

10 % Acetoxyaceton-Dosierung: Ein Reaktionsansatz war zusammengesetzt aus 10 ml (10,8 g) Acetoxyaceton, 20 ml (15,7 g) Isopropanol, 40 ml KPi-Puffer, pH 7,0, 30 ml T-ADH Enzym (6.000 U T-ADH, von der Firma Jülich Fine Chemicals GmbH bezogen) und 50 µM NADP. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 91 %. Der Enantiomerenüberschuss ee des Produktes (S)-1-Acetoxy-2-Propanol betrug 100 %.

20 % Acetoxyaceton-Dosierung: Ein Reaktionsansatz war zusammengesetzt aus 20 ml (21,5 g) Acetoxyaceton, 30 ml (23,6 g) Isopropanol, 5 ml KPi-Puffer, pH 7,0, 45 ml T-ADH Enzym (9.000 U T-ADH, von der Firma Jülich Fine Chemicals GmbH bezogen) und 50 µM NADP. Der Reaktionsansatz wurde bei 30°C gerührt. Zu verschiedenen Zeitpunkten wurden 0,1 ml Proben des Reaktionsansatzes genommen, mit 1 ml MTBE extrahiert und durch chirale GC (siehe 3. Beispiel) analysiert. Nach 24 h betrug der Reaktionsumsatz des eingesetzten Acetoxyacetons 36,3 %. Der Enantiomerenüberschuss ee des Produktes (S)-1-Acetoxy-2-Propanol betrug 100 %.

## Patentansprüche

1. Verfahren zur Herstellung einer chiralen Verbindung der Formel (I), 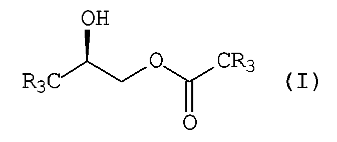 wobei R gleich oder verschieden ist und H, oder organischer Rest bedeutet, bei dem eine Biotransformationszusammensetzung, umfassend eine Verbindung der Formel (II), 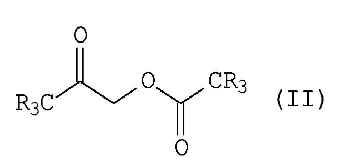 wobei R die genannte Bedeutung hat, eine Oxidoreduktase, einen Redox-Cofaktor und ein Cosubstrat, zur Reaktion gebracht wird, wobei die chirale Verbindung der Formel (I) entsteht und nachfolgend isoliert wird.

2. Verfahren gemäß Anspruch 1, **dadurch gekennzeichnet, dass** R gleich oder verschieden ist und H oder C₁-C₂₀-Alkyl-, C₂-C₂₀₋Alkenyl-, C₃-C₈-Cycloalkyl-, C₆-C₂₀-Aryl- oder C₅-C₂₀-Heteroaryl-, bei dem ein oder mehrere C-Atome durch Atome, ausgewählt aus der Gruppe B, N, O, Si, P und S, ersetzt sein können oder bei dem ein oder mehrere C-Atome substituiert sein können durch F, Cl, Br, J, C₃-C₈-Cycloalkyl, C₆-C₂₀-Aryl, C₅-C₂₀-Heteroaryl, CN, NH₂, NO oder NO₂, bedeutet.

3. Verfahren gemäß Anspruch 1, **dadurch gekennzeichnet, dass** R gleich oder verschieden ist und H oder C₁-C₂₀-Alkyl-, C₃-C₈₋Cycloalkyl-, C₆-C₂₀-Aryl- oder C₅-C₂₀-Heteroaryl-, bei dem ein oder mehrere C-Atome durch F, Cl, C₃-C₈-Cycloalkyl, C₆-C₂₀-Aryl oder C₅-C₂₀-Heteroaryl substituiert sein können, bedeutet.

4. Verfahren gemäß Anspruch 1, **dadurch gekennzeichnet, dass** R H bedeutet.

5. Verfahren gemäß einem der Ansprüche 1 bis 4, **dadurch gekennzeichnet, dass** die Oxidoreduktase eine Carbonylreduktase mit R-Spezifität ist.

6. Verfahren gemäß Anspruch 5, **dadurch gekennzeichnet, dass** als R-spezifische Carbonylreduktase eine sekundäre ADH oder eine Fettsäuresynthetase eingesetzt wird.

7. Verfahren gemäß Anspruch 5 oder 6, **dadurch gekennzeichnet, dass** als R-spezifische Carbonylreduktase eine LB-ADH aus Lactobacillus brevis eingesetzt wird.

8. Verfahren gemäß einem der Ansprüche 1 bis 7, **dadurch gekennzeichnet, dass** der Redox-Cofaktor ausgewählt ist aus Verbindungen der Gruppe NAD, NADP, NADH, NADPH und deren Salzen.

9. Verfahren gemäß einem der Ansprüche 1 bis 8, **dadurch gekennzeichnet, dass** das Cosubstrat eine Verbindung ist, die als Reduktionsmittel enzymatisch oxidiert wird, wobei die dabei gewonnenen Elektronen auf NAD bzw. NADP übertragen werden und somit NADH bzw. NADPH regeneriert wird.

10. Verfahren gemäß einem der Ansprüche 1 bis 9, **dadurch gekennzeichnet, dass** die Carbonylreduktase eine Alkohldehydrogenase ist und das Cosubstrat ein Alkohol ist.

11. Verfahren gemäß Anspruch 10, **dadurch gekennzeichnet, dass**, der Alkohol Isopropanol oder 2-Butanol ist.

12. Verfahren gemäß Anspruch 1 bis 11, **dadurch gekennzeichnet, dass** Edukte der allgemeinen Formel (II) zu > 80 %, bevorzugt > 90 %, insbesondere bevorzugt > 93 % zu einer Verbindung der allgemeinen Formel (I) umgesetzt werden.

13. Verfahren gemäß Anspruch 1 bis 12, **dadurch gekennzeichnet, dass** die Verbindung der allgemeinen Formel (I) aus dem Reaktionsansatz mittels eines mit Wasser nicht mischbaren, organischen Lösungsmittels oder durch Destillation extrahiert wird.

14. Biotransformationszusammensetzung umfassend Fermenterzellen enthaltend ein CR-Enzym, eine Verbindung der Formel (II), einen Redox-Cofaktor ausgewählt aus den Verbindungen NAD, NADH, NADP, NADPH, und deren Salzen, eine Cosubstrat ausgewählt aus der Gruppe Isopropanol, 2-Butanol und Glucose sowie im Falle von Glucose als Cosubstrat eine GDH als Cofaktor-regenerierendes Enzym.

15. Zusammensetzung gemäß Anspruch 14 enthaltend zwischen 1 %(v/v) und 40 % (v/v) bezogen auf den Gesamtansatz Fermentationsmedium mit Fermenterzellen mit einem Biomasseanteil von 0,05 - 2 % (w/v) enthaltend ein CR-Enzym, eine Verbindung der Formel (II) in einer Menge von 10 %(w/v) bis 60 % (w/v) des Gesamtansatzes und zwischen 10 % (w/v) und 50 % (w/v) bezogen auf den Gesamtansatz ein Cosubstrat ausgewählt aus der Gruppe Isopropanol und 2-Butanol und den Redox-Cofaktor in einer Menge zwischen 10 µM und 200 µM.

16. Zusammensetzung gemäß Anspruch 14 oder 15,**dadurch gekennzeichnet, dass** das CR-Enzym eine ADH ist.

17. Zusammensetzung gemäß einem der Ansprüche 14 bis 16, **dadurch gekennzeichnet, dass** die Verbindung der Formel (II) Acetoxyaceton (R = H) ist.

18. Verbindung der allgemeinen Formel (I), wobei R gleich H ist ((R)-1-Acetoxy-2-Propanol).
